# EUROPEAN PATENT SPECIFICATION

(11) **EP 3 366 703 B1**
(45) Date of publication and mention of the grant of the patent: **03.04.2019**
(21) Application number: 17158412.1
(22) Date of filing: 28.02.2017
(51) Int. Cl.: C07K 16/28, A61K 41/00, C07K 14/55

(54) **IMMUNE CHECKPOINT THERAPY WITH HYPERTHERMIA**
IMMUN KONTROLLPUNKT THERAPIE MIT HYPERTHERMIE
THÉRAPIE CIBLANT LES POINTS DE CONTRÔLE IMMUNITAIRES AVEC HYPERTHERMIE

(43) Date of publication of application: 29.08.2018
(73) Proprietor: Kleef, Ralf, 1130 Vienna (AT)
(72) Inventor: Kleef, Ralf, 1130 Vienna (AT)
(74) Representative: Sonn & Partner Patentanwälte

(56) References cited:
- WO-A1-00/28813
- RALF KLEEF: "Near complete remission of pulmonary metastases in triple negative breast cancer (TNBC) using low-dose immune checkpoint (IC) inhibitors with high dose (HD) interleukin-2 (IL-2) and fever range hyperthermia. (Abstract e33111)", JOURNAL OF CLINICAL ONCOLOGY, vol. 34, no. 15, 20 May 2016 (2016-05-20), XP002771488,
- Tenho Hietanen ET AL: "Restoring Natural Killer Cell Cytotoxicity After Hyperthermia Alone or Combined with Radiotherapy", Anticancer research, 1 February 2016 (2016-02-01), page 555, XP55441396, Greece Retrieved from the Internet: URL:http://ar.iiarjournals.org/content/36/ 2/555.full [retrieved on 2018-01-16]
- E. A. Repasky ET AL: "Temperature Matters! And Why It Should Matter to Tumor Immunologists", CANCER IMMUNOLOGY RESEARCH, vol. 1, no. 4, 1 October 2013 (2013-10-01) , pages 210-216, XP55441400, US ISSN: 2326-6066, DOI: 10.1158/2326-6066.CIR-13-0118

## Description

The present invention relates to the treatment of cancer patients with immune checkpoint inhibitors.

Cancer is associated with global immune suppression of the host. Malignancy-induced immune suppressive effect can be circumvented by blocking different immune checkpoints and tip the immune balance in favour of immune stimulation and unleash cytotoxic effects on cancer cells. Human antibodies directed against immune checkpoint proteins: cytotoxic T lymphocytes antigen-4 (CTLA-4) and programmed death-1 (PD-1), programmed death-ligand 1 (PD-L1), have shown therapeutic efficacy in advanced melanoma and non-small-cell lung cancer and other malignancies. Immune check point blockade antibodies lead to diminished tolerance to self and enhanced immune ability to recognize and eliminate cancer cells. As a class these agents have immune-related adverse events i.a. due to decreased ability of effector immune cells to discriminate between self and non-self (Wolchok et al., N. Eng. J. Med. 369 (2013), 122-133; Callahan et al., Front. Oncol. 4 (2015), article 385; Das et al., J. Immunol. 194 (2015, 950-959; Khan et al., J. Oncology (2015); article ID 847383; Naidoo et al., Ann. Oncol. 26 (2015), 2375-2391).

Despite the significant improvement of this type of immune therapy, there are still significant problems associated with this therapy, specifically with respect to efficacy/safety issues and (auto-)immune-related adverse effects associated with such checkpoint inhibitor (CI) therapies.

It is the primary object of the present invention to provide new and improved therapies for cancer patients based on CIs, especially therapies with higher efficacy and/or lower adverse effects. A secondary objective of the present invention is to offer effective immunotherapies which are much less expensive than the currently established therapy regimens (The checkpoint inhibitor drugs are expected to become a $30bn-plus market by 2022) .

Therefore, the present invention relates to (a) an antibody or an antigen-binding portion thereof that specifically binds to and inhibits Programmed Death-1 (PD-1) and/or an antibody or an antigen-binding protein portion thereof that specifically binds to and inhibits Programmed Death-L1 (PD-L1); (b) an antibody or an antigen-binding portion thereof that specifically binds to and inhibits Cytotoxic T-Lymphocyte Antigen-4 (CTLA-4); and (c) Interleukin-2 (IL-2) for the combined use in the treatment of a cancer patient,
wherein the body core temperature of said patient is kept at a temperature of 39.0°C to 40.5°C, preferably of 39.5°C to 40.5°C for at least 5 h per day for at least 5 consecutive days by a low dose (LD) IL-2 treatment, wherein the overall amount of IL-2 administered is in the range of 40 to 70 million units IL-2/week.

The present invention provides a CI therapy wherein a PD-1/PD-L1 inhibiting agent (preferably an PD-1/PD-L1 antibody or a PD-1/PD-L1 binding portion thereof) and a CTLA-4 inhibiting agent (preferably a CTLA-4 antibody or a CTLA-4 binding portion thereof) is administered to a patient, preferably in a lower dose than the doses currently applied (see e.g. Wolchok et al., 2015), together with IL-2, wherein the IL-2 administration is not accompanied with antipyretics (as required by the administration leaflets) but wherein the patient is allowed to develop controlled significant fever (i.e. at least 5 h over 39.0°C, preferably over 39.5°C for at least 5 consecutive days).

The presented concept and invention targeting the induction of fever in cancer patients is a modern adaptation of a concept for immunotherapy of cancer that started 130 years ago with William Coley (Kleef et al. Neuroimmunomodulation. 2001;9(2):55-64. Review.) and that has been adapted with surprising effectiveness to modern immunotherapy by the present invention.

Whereas CI therapies have applied IL-2 in the past (Kleef et al., ASCO (2016), Abstract 166013 and J. Clin. Oncol. 34, 2016, abstract e33111; Wolchok et al., 2015; Khan et al., 2015), fever was induced mainly by whole body or local hyperthermia devices with 13.56 MHz modulated radiofrequency (e.g. Oncotherm, Synchrotherm, etc.). Such methods are also applied in connection with chemotherapy and radiation therapy in cancer patients. Although such hyperthermia treatments have been successfully applied (and are even preferably applied in the methods according to the present invention in addition to the basic fever treatment according to the present invention), the methods and treatments according to the present invention have shown significantly better success rates by imposing a longer (at least 5 consecutive days instead of (only) one radiofrequency treatment or more than one radiofrequency treatment timely separated) and more intense fever induction (at least 5 h per day, preferably at least 6 h per day, more preferred at least 7 h per day, especially at least 8 h per day) for at least five consecutive days) on the patient. It is also possible - if the patient's condition allows - to keep linger fever durations per day (at least 10 h or at least 12 h), however, such longer circles have not been proven yet to gain additional benefit and have also practical drawback, since the patients also need to recover from the fever circles. Accordingly, the fever treatments should also be optimised within the borderlines of the present invention to take the individual condition of the patient before and during the treatment into consideration.

The daily fever induction is necessary to be performed at least for five consecutive days. It is also possible to perform the induction of at least 39.0°C, preferably of 39.5°C to 40.5°C, per diem for at least 6 consecutive days, preferably for at least 7 consecutive days, especially for at least 8 consecutive days (e.g. for 9, 10, 11, or 12 consecutive days).

It is important according to the present invention to keep the patients at a temperature level of at least 39.0°C, preferably of 39.5°C to 40.5°C, i.e. at high fever, to achieve the results according to the present invention. Lower temperatures or less duration (e.g. two or three days only) will not achieve the efficacy of the present invention. Higher fever (up to 41.0°C) is in principle not excluded, however, care should be taken that the patient is kept in a condition wherein the fever is not life-threatening. This is why the aimed range of preferably 39.0°C to 40.5°C has shown to be the balanced range with respect to risk/efficacy for the present invention.

The fever according to the present invention is preferably imposed on the patient by administering IL-2 in a way so as to achieve the aimed temperature range. The patients may therefore be "IL-2 titrated" with respect to the fever, i.e. the fever may be induced and controlled simply by the IL-2 dosage, however, without co-administration of fever-reducing agents (antipyretics) which are usually required as IL-2 co-medication (see product leaflet for the commercial IL-2 product PROLEUKIN® (aldesleukin) recommending standard antipyretic therapy (especially NSAID therapy immediately before IL-2 therapy as concomitant medications; see also e.g. Dutcher et al., J. Immunother. Cancer 2 (2014), 26). According to a preferred embodiment of the present invention, body temperature of the patient is controlled by administration of IL-2 ("IL-2 fever titration"; "IL-2 induced fever").

The present invention preferably applies the IL-2 treatment with lower doses than usually recommended, especially in connection with CI therapy: Whereas usual IL-2 administration applies in total well over 500 million units IL-2/week, the overall amount of IL-2 administered in the course of the present invention is in the range of 40 to 70 million units IL-2/week, especially in the range of 45 to 60 million units IL-2.

According to a preferred embodiment, IL-2 is administered by continuous administration of 100.000 to 1.000.000 units/kg body weight, preferably 400.000 to 720.000 units/kg body weight, especially as an initial induction of the fever according to the present invention. The following repeated fever cycles can be safeguarded ("titrated") with lower IL-2 doses, depending on the response of the patient.

Consequently, it is preferred to omit any antipyretic medication in the course of the IL-2 administration according to the present invention or at least reduce such medication to not impede the development of fever in the desired temperature range. Therefore, a preferred embodiment of the present invention involves a treatment which does not include administration of an antipyretic, especially a non-steroidal anti-inflammatory drug (NSAID).

The CI therapy according to the present invention may be performed by any suitable CTLA-4/(PD-1/PD-L1) inhibitor couple. Such inhibitors are widely available in the present field.

For example, WO 2013/173223 A1 discloses antibodies ("Abs") specific for (and inhibiting) PD-1/PD-L1 and CTLA-4 (see also e.g. US 8, 008, 449 B2 and US 7,943,743 B2).

Preferred anti-PD-1/PD-L1 HuMAbs exhibit one or more of the following characteristics: (a) binds to human PD-1 with a KD of 1 x 10-7 M or less, as determined by surface plasmon resonance using a Biacore biosensor system; (b) does not substantially bind to human CD28, CTLA-4 or ICOS; (c) increases T-cell proliferation in a Mixed Lymphocyte Reaction (MLR) assay; (d) increases interferon-γ production in an MLR assay; (e) increases IL-2 secretion in an MLR assay; (f) binds to human PD-1 and cynomol-gus monkey PD-1 ; (g) inhibits the binding of PD-L1 and/or PD-L2 to PD-1 ; (h) stimulates antigen-specific memory responses; (i) stimulates Ab responses; and (j) inhibits tumour cell growth in vivo. Anti- PD-1 Abs of the present invention include mAbs that bind specifically to human PD-1 and exhibit at least one, preferably at least five, of the preceding characteristics. US 8,008,449 B2 exemplifies seven anti-PD-1 HuMAbs: 17D8, 2D3, 4H1, 5C4 (also referred to herein as nivolumab or BMS-936558), 4A1 1, 7D3 and 5F4. Isolated DNA molecules encoding the heavy and light chain variable regions of these Abs have been sequenced, from which the amino acid sequences of the variable regions were deduced and disclosed e.g. in WO 2013/173223 A1. Preferred PD-1 inhibitors according to the present invention are mainly nivolumab (Opdivo), and also pembrozilumab (Keytruda), avelumab and atezolizumab (Tecentric).

Preferred anti-CTLA-4 antibodies of the present invention can bind to an epitope on human CTLA-4 so as to inhibit CTLA-4 from interacting with a human B7 counter receptor. Because interaction of human CTLA-4 with human B7 transduces a signal leading to inactivation of T-cells bearing the human CTLA-4 receptor, antagonism of the interaction effectively induces, augments or prolongs the activation of T cells bearing the human CTLA-4 receptor, thereby prolonging or augmenting an immune response. Anti-CTLA-4 antibodies are described e.g. in WO 01/14424 A and WO 00/37504 A; An exemplary clinical anti-CTLA-4 antibody is human monoclonal antibody ipilimumab (WO 2013/173223 A1; Hodi et al., N. Eng. J. Med. 363 (2010), 711 to 723; Wolchok et al., 2015). A further example is tremelimumab. An overview over further suitable anti-CTLA-4 antibodies for use in the therapy according to the present invention is disclosed in US 8,008,449 B2.

Preferably, the anti-CTLA-4 antibody binds to human CTLA-4 with a KD of 5×10-8 M or less, binds to human CTLA-4 with a KD of 1×10-8 M or less, binds to human CTLA-4 with a KD of 5×10-9 M or less, or binds to human CTLA-4 with a KD of between 1×10-8 M and 1×10-10 M or less.

In contrast to the high doses of antibodies which are usually applied for CI with combined anti-PD-1 and anti-CTLA-4 inhibitors or antibodies (see e.g. Wolchok et al., 2015), it is preferred according to the present invention to use lower doses of such antibodies.

Accordingly, a preferred embodiment of the present invention applies a CI therapy with each antibody (PD-1, CTLA-4) being administered at a dosage ranging from 0.05 to 1 mg/kg body weight, preferably from 0.1 to 0.8 mg/kg body weight, especially from 0.3 to 0.5 mg/kg body weight, at least once a week, for at least two weeks, preferably for at least three weeks, especially at least four weeks.

Preferably, the PD-1 antibody is administered in slightly higher doses than the CTLA-4 antibody, especially in the case of nivolumab as PD-1 antibody and ipilimumab as CTLA-4 antibody. Accordingly, the following dosages of PD-1 antibody/CTLA-4 antibody, especially nivolumab/ipilimumab are specifically preferred: 0.1 to 1 mg/kg PD-1 with 0.05 to 0.8 mg/kg; CTLA-4, even more preferred 0.3 to 0.7 mg/kg PD-1 with 0.1 to 0.5 mg/kg CTLA-4, especially 0.4 to 0.6 mg/kg PD-1 with 0.2 to 0.4 mg/kg CTLA-4.

According to another preferred embodiment, cyclophosphamide is administered additionally to the patient in the course of the present invention, preferably in an amount of 100 to 500 mg/m², especially in an amount of 200 to 400 mg/m². Appropriate dosages and amounts of cyclophosphamide may be individually determined based on the individual patient to effectively down modulate T_{reg} cells in this patient at the relevant stage of therapy.

In addition, also antimicrobial agents, especially taurolidine, may preferably be administered additionally to said patient.

According to another aspect, the present invention refers to a(ny) checkpoint therapy (CT; CI therapies) for use in a tumour patient, wherein the body core temperature of said patient is kept at a temperature of 39.0°C to 40.5°C, preferably of 39.5°C to 40.5°C for at least 5 h per day for at least 5 consecutive days, wherein the overall amount of IL-2 administered is in the range of 40 to 70 million units IL-2/week. The therapy principle according to the present invention, although proven to be successful in the treatment of combined inhibition of PD-1 and CTLA-4 (i.e. wherein PD-1 and CTLA-4 inhibitors, especially antibodies, are administered in combination), is also applicable to all treatment regimen wherein immune checkpoints are addressed (inhibited), i.e. which target regulatory pathways in T cells to enhance antitumor immune responses. Practical examples for such CI therapies wherein the principle of the present invention can be applied are disclosed e.g. in Topailan et al., Cancer Cell 27 (2015), 450-461). Antibodies used in the course of such CI therapies and wherein the present invention can be included in the therapy schemes with such antibodies or combination of antibodies are nivolumab (Opdivo, BMS), and ipilimumab (Yervoy, BMS), as disclosed above, but also other antibodies being specific for (inhibiting) immune checkpoint proteins, such pembrolizumab (Keytruda, MK-3475, Merck), pidilizumab (CT-011, Cure Tech), BMS-936559 (anti-PD-1 receptor), atezolizumab (MPDL3280A, Roche), avelumab (Merck KGaA, Darmstadt, Germany & Pfizer) (anti-PD-L1 receptor), and/or tremelimumab (anti CTLA-4) (see also: Ott et al., J. Immunother. Cancer 5 (2017), 16).

The present invention is further described by the following examples and the figures, yet without being restricted thereto.
Fig. 1A shows the body core temperature of the cancer patient of case 1; Figs. 1B and 1C show endoscopies before immunotherapy (month 0) demonstrating exulcerated advanced T4 esophageal cancer; Fig. 1D and 1E show: endoscopy following immunotherapy (month 2) according to the present invention demonstrating complete remission.
Fig. 2 shows the body core temperature of the cancer patient of case 2.
Fig. 3 shows the body core temperature of the cancer patient of case 3.

### EXAMPLES:

Further case reports referring to Immunotherapy in stage IV cancer patients using moderate dosed IL-2 for fever induction in combination with low-dose checkpoint inhibitors and hyperthermia

### CASE 1: Complete response of stage IIIB esophageal cancer combining low-dose checkpoint inhibitors with interleukin-2 (IL-2) and fever range hyperthermia.

Advanced stage inoperable esophageal cancer has a poor prognosis and patients rarely enjoy durable complete response to treatment; progression free survival often is limited.

### Materials and methods:

The patient was a 56-year-old male newly diagnosed with adenocarcinoma of the esophagus with mediastinal lymphadenopathy. Histology revealed adenocarcinoma stage UICC IIIB T4 N2 with disseminated mediastinal, para-esophageal and cervical lymph node metastasis measuring up to 2.2 cm. HER-2/new score was positive. The patient refused suggested neoadjuvant CHT with FLOT. Clinically the patient presented with Karnofsky index of 90% with increasing difficulties swallowing solid food and rapid weight loss of 6 kg in the last 2 months.

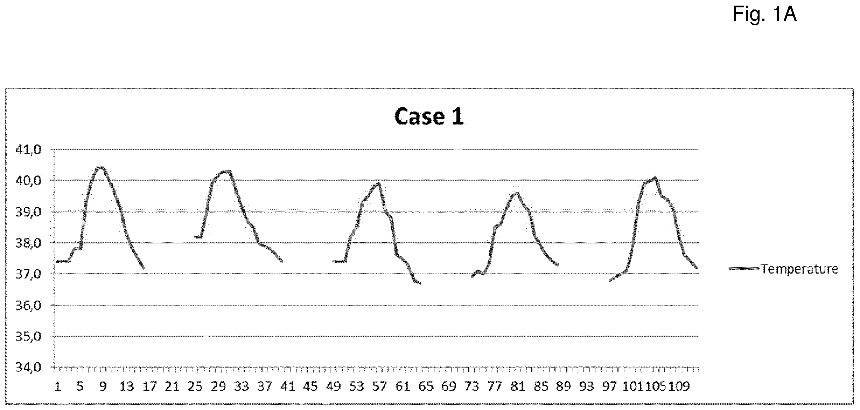

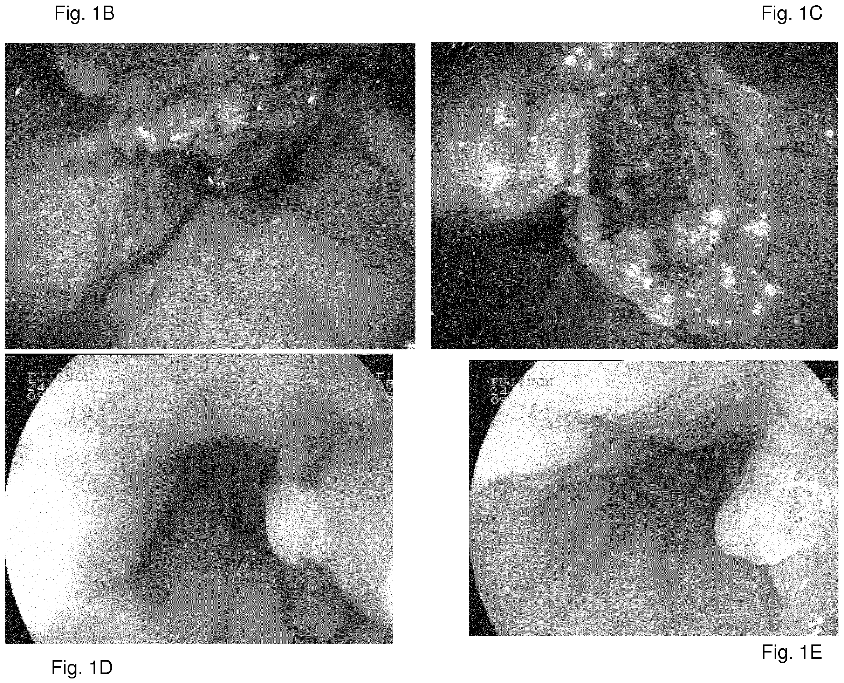

Therapy started consisted of administration of the following combination protocol: Low-dose PD-1 immune checkpoint (IC) inhibitor nivolumab (0.5 mg/kg) with CTLA-4 IC inhibitor ipilimumab (0.3 mg/kg) administered weekly, over three weeks. This was accompanied by loco regional hyperthermia with radiofrequency fields (13.56 MHz) using the Syncrotherm device 3 times per week (max output 400 w) over the tumor region in combination with high dose vitamin C (0.5 g/kg) and alpha lipoic acid (600mg) over three weeks. This was followed by long duration fever range whole body hyperthermia (using the Heckel device) in combination with low dose chemotherapy using cyclophosphamide 300 mg/m² to down modulate T_{reg} cells. Next, moderate dose i.v. interleukin 2 (IL-2) under Taurolidine protection was administered for five days with careful titration to daily fever hyperthermia of max 39.5°C. Herceptin in a dosage of only 4 mg/kg (patient refused higher dosage) was administered once. The body core temperature of the cancer patient during the treatment of the present invention is depicted in Fig. 1.

### Results:

Unexpectedly, restaging 8 weeks following initiation of therapy with Gastroesophagoscopy (Upper GI Endoscopy) revealed complete response. This was confirmed by histological analysis of multiple biopsies in the former tumour bed confirming complete pathological response. At that time the patient had started gaining weight again and was free of any cancer-related symptoms. Several months later, he has regained 6 kg of weight, feels good, has no dysphagia, and continues to be monitored. Figs. 1B and 1C show endoscopies before immuno-therapy (month 0) demonstrating exulcerated advanced T4 esophageal cancer; Fig. 1D and 1E show: endoscopy following immunotherapy (month 2) according to the present invention demonstrating complete remission.

### Conclusion:

This advanced stage cancer patient therefore had a surprising and completely unexpected complete response to primary immunotherapy treatment.

### CASE 2: Complete clinical remission of stage IV inoperable prostate cancer combining low-dose checkpoint inhibitors with interleukin-2 (IL-2) and fever range hyperthermia

Inoperable stage IV advanced prostate cancer following R1 resection has a poor prognosis.

The patient was a 58 old year old male first diagnosed 07/2016 when he underwent radical prostatectomy and lymphadenectomy on August 23, 2016 in Germany for locally advanced prostate cancer with infiltration of the sphincter muscle pN1 (3/13), G3, R1 Gleason score 9, ISUP Grading WHO: 5. Initially the patient had severe obstructive urine flow and needed urgent treatment. Radiotherapy and permanent catheter was not consented.

One month post-operative MRI restaging of abdomen and small pelvis from 20.09.2016 demonstrated a lesion left posterior of the bladder neck of 1.3×1.2×2 cm inseparable from the left lateral rectal wall and adjoining portion of pubococcegeus portion of the left levator ani. This soft tissue mass well explained the severe small pelvis pain the patient was suffering from.

The suggested palliative hormonal therapy and radiation was not initiated yet. Radical resection including sphincter resection with permanent colostomy was rejected.

ND: 10 years ago the patient was diagnosed with hepatitis C which was cured 2013 by triple combination therapy of ribarafin, interferon and sovalvir.

The patient presented with Karnofsky Index of 80%, left sided deep pain in the small pelvis VAS9 (!) treated with NSAR, severe incontinence. Weight loss of 10 kg following surgery.

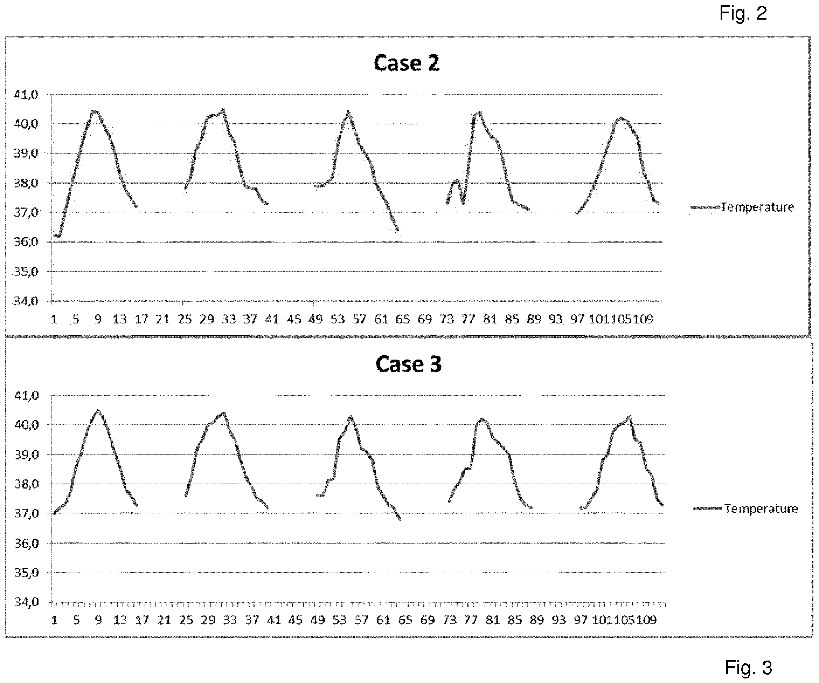

The patient underwent the same treatment concept as described in detail in case #1 with the addition of hormone therapy with Trenantone/Casodex and metronomic low-dose chemotherapy and without Herceptin. The body core temperature of the cancer patient during the treatment of the present invention is depicted in Fig. 2.

### Results:

Unexpectedly restaging with MRI of the abdomen and small pelvis at the end of February 2017 revealed complete remission; the patient is in excellent health and has no tumor-associated symptoms or ailments.

### CASE 3: Complete clinical remission of stage IV colon cancer combining low-dose checkpoint inhibitors with interleukin-2 (IL-2) and fever range hyperthermia

Metastatic stage IV colon cancer has a poor prognosis with very limited overall survival and limited therapeutic options.

The patient was a 45 year old female first diagnosed with metastatic colon cancer in 05/2013 cT3 cN2 cMlb (liver and lung). Histology revealed ulcerated low differentiated adenocarcinoma of enteral type, K-RAS mutation exon 2: Substitution of p.G12V, no microsatellite instability. Further staging proved locally advanced subtotal proximal rectal cancer with multiple local regional lymph node metastases, liver metastasis and disseminated lung metastasis of up to 3.5 cm.

The patient underwent multiple palliative systemic chemotherapies with FOLFOX and Avastin which induced partial remission followed by Avastin maintenance therapy. PET staging in 05/2015 showed PD in the rectal area as well as pulmonic PD but complete remission of liver metastasis. 06-07/2015 the patient underwent surgical resection of the primary colon cancer with transient colostomy. 09/2015 massive progression of lung metastasis was diagnosed.

The patient presented with Karnofsky Index of 90%, mild shortness of breath on exertion.

The patient underwent the same treatment concept as described in detail in case #1. The body core temperature of the cancer patient during the treatment of the present invention is depicted in Fig. 3.

Interestingly, initial restaging 5 months later indicated progressive disease of 2 lung metastasis which were clinically presenting with increased coughing. The patient therefore underwent radiation therapy to the pulmonic-mediastinal area.

### Results:

Unexpectedly restaging with CT of the thorax and abdomen at the end of June 2016 revealed complete remission of previously described progressive (DD: pseudo-progression) lung metastasis; the patient is in excellent health and has no tumor-associated symptoms or ailments.

### Conclusion:

The intermittent PD of the patient's lung metastasis 5 months following our treatment concept may be interpreted as so-called pseudo-progression. It is realized by now that immune therapies exert their effects on cancer indirectly by building an immune response first, which is then followed by changes in tumor burden or patient survival. The median time to achieve complete response was 30 months. Immune therapy may induce unusual kinetics of antitumor response, which is not captured by Response Evaluation Criteria in Solid Tumors (RECIST) or World Health Organization criteria (Postow et al., J. Clin. Oncol. 33 (2015), 1974-1982).

## Claims

1. An (a) antibody that specifically binds to and inhibits Programmed Death-1 (PD-1) and/or an antibody that specifically binds to and inhibits Programmed Death-L1 (PD-L1); (b) an antibody that specifically binds to and inhibits Cytotoxic T-Lymphocyte Antigen-4 (CTLA-4); and (c) Interleukin-2 (IL-2) for the combined use in the treatment of a cancer patient, wherein the body core temperature of said patient is kept at a temperature of 39.0°C to 40.5°C, preferably of 39.5°C to 40.5°C for at least 5 h per day for at least 5 consecutive days by a low dose (LD) IL-2 treatment, wherein the overall amount of IL-2 administered is in the range of 40 to 70 million units IL-2/week.

2. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to claim 1, wherein the body core temperature of said patient is kept at a temperature of 39.0°C to 40.5°C, preferably of 39.5°C to 40.5°C for at least 5 h per day, preferably at least 6 h per day, preferably at least 7 h per day, especially at least 8 h per day, for at least 5 consecutive days.

3. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to claim 1 or 2, wherein the body core temperature of said patient is kept at a temperature of 39.0°C to 40.5°C, preferably of 39.5°C to 40.5°C for at least 5 h per day for at least 6 consecutive days, preferably for at least 7 consecutive days, especially for at least 8 consecutive days.

4. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to any one of claims 1 to 3, wherein the body temperature of said patient is controlled by administration of IL-2.

5. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to any one of claims 1 to 4, wherein the treatment does not include administration of an antipyretic, especially a non-steroidal anti-inflammatory drug (NSAID).

6. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to any one of claims 1 to 5, wherein IL-2 is administered by continuous administration of 100.000 to 1.000.000 units/kg body weight, preferably 400.000 to 720.000 units/kg body weight.

7. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to any one of claims 1 to 6, wherein, independently of each other, each antibody is administered at a dosage ranging from 0.05 to 1 mg/kg body weight, preferably from 0.1 to 0.8 mg/kg body weight, especially from 0.3 to 0.5 mg/kg body weight, at least once a week, preferably at least twice a week, especially at least three times a week, for at least two weeks, preferably for at least three weeks, especially at least four weeks.

8. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to any one of claims 1 to 7, wherein cyclophosphamide is administered additionally to said patient, preferably in an amount of 100 to 500 mg/m², especially in an amount of 200 to 400 mg/m².

9. An (a) antibody that specifically binds to and inhibits PD-1 and/or an antibody that specifically binds to and inhibits PD-L1; (b) an antibody that specifically binds to and inhibits CTLA-4; and (c) IL-2 for the combined use according to any one of claims 1 to 8, wherein taurolidine is administered additionally to said patient.

## Patentansprüche

1. (a) Antikörper, der spezifisch an PD-1 (Programmed Death-1) bindet und dieses inhibiert und/oder Antikörper, der spezifisch an PD-L1 (Programmed Death-L1) bindet und dieses inhibiert; (b) Antikörper, der spezifisch an CTLA-4 (Cytotoxic T-Lymphocyte Antigen-4) bindet und dieses inhibiert; und (c) Interleukin-2 (IL-2) zur kombinierten Anwendung bei der Behandlung eines Krebspatienten, wobei die Körperkerntemperatur des Patienten an mindestens 5 aufeinanderfolgenden Tagen anhand einer niedrig dosierten (LD = *low dose*) Behandlung mit IL-2 für mindestens 5 h pro Tag auf einer Temperatur von 39,0 °C bis 40,5 °C, vorzugsweise von 39,5 °C bis 40,5 °C, gehalten wird, wobei die verabreichte Gesamtmenge an IL-2 in einem Bereich von 40 bis 70 Millionen Einheiten an IL-2 pro Woche liegt.

2. (a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach Anspruch 1, wobei die Körperkerntemperatur des Patienten an mindestens 5 aufeinanderfolgenden Tagen für mindestens 5 h pro Tag, vorzugsweise für mindestens 6 h pro Tag, vorzugsweise für mindestens 7 h pro Tag, insbesondere für mindestens 8 h pro Tag, auf einer Temperatur von 39,0 °C bis 40,5 °C, vorzugsweise von 39,5 °C bis 40,5 °C, gehalten wird.

3. (a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach Anspruch 1 oder 2, wobei die Körperkerntemperatur des Patienten an mindestens 6 aufeinanderfolgenden Tagen, vorzugsweise an mindestens 7 aufeinanderfolgenden Tagen, insbesondere an mindestens 8 aufeinanderfolgenden Tagen, für mindestens 5 h pro Tag auf einer Temperatur von 39,0 °C bis 40,5 °C, vorzugsweise von 39,5 °C bis 40,5 °C, gehalten wird.

4. a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach einem der Ansprüche 1 bis 3, wobei die Körpertemperatur des Patienten anhand der Verabreichung von IL-2 reguliert wird.

5. a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach einem der Ansprüche 1 bis 4, wobei die Behandlung keine Verabreichung eines Antipyretikums, insbesondere eines nichtsteroidalen, antiinflammatorischen Wirkstoffs (NSAID = *non-steroidal anti-inflammatory drug*) einschließt.

6. a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach einem der Ansprüche 1 bis 5, wobei die Verabreichung von IL-2 anhand einer kontinuierlichen Verabreichung von 100.000 bis 1.000.000 Einheiten/kg Körpergewicht, vorzugsweise von 400.000 bis 720.000 Einheiten/kg Körpergewicht, erfolgt.

7. a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach einem der Ansprüche 1 bis 6, wobei jeder der Antikörper jeweils unabhängig in einer Dosierung verabreicht wird, die in einem Bereich von 0,05 bis 1 mg/kg Körpergewicht, vorzugsweise von 0,1 bis 0,8 mg/kg Körpergewicht, insbesondere von 0,3 bis 0,5 mg/kg Körpergewicht, liegt, und zwar mindestens ein Mal pro Woche, vorzugsweise mindestens zwei Mal pro Woche, insbesondere mindestens drei Mal pro Woche, für mindestens zwei Wochen, vorzugsweise für mindestens drei Wochen, insbesondere für mindestens vier Wochen.

8. (a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach einem der Ansprüche 1 bis 7, wobei dem Patienten zusätzlich Cyclophosphamid verabreicht wird, und zwar vorzugsweise in einer Menge von 100 bis 500 mg/m², insbesondere in einer Menge von 200 bis 400 mg/m².

9. (a) Antikörper, der spezifisch an PD-1 bindet und dieses inhibiert und/oder Antikörper, der spezifisch an PD-L1 bindet und dieses inhibiert; (b) Antikörper, der spezifisch an CTLA-4 bindet und dieses inhibiert; und (c) IL-2 zur kombinierten Anwendung nach einem der Ansprüche 1 bis 8, wobei dem Patienten zusätzlich Taurolidin verabreicht wird.

## Revendications

1. (a) Anticorps qui se lie spécifiquement à et inhibe PD-1 (Programmed Death-1) et/ou anticorps qui se lie spécifiquement à et inhibe PD-L1 (Programmed Death-L1) ; (b) anticorps qui se lie spécifiquement à et inhibe CTLA-4 (Cytotoxic T-Lymphocyte Antigen-4) ; et (c) interleukine-2 (IL-2) pour une utilisation combinée dans le traitement d'un patient cancéreux, où la température corporelle centrale dudit patient est maintenue à une température comprise entre 39 °C et 40,5 °C, de préférence entre 39,5 °C et 40,5 °C, pendant au moins 5 h par jour pendant au moins 5 jours consécutifs par un traitement par l'IL-2 à faible dose (LD), où la quantité globale d'IL-2 administrée est comprise dans la plage de 40 à 70 millions d'unités d'IL-2/semaine.

2. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon la revendication 1, où la température corporelle centrale dudit patient est maintenue à une température comprise entre 39 °C et 40,5 °C, de préférence entre 39,5 °C et 40,5 °C, pendant au moins 5 h par jour, de préférence au moins 6 h par jour, de préférence au moins 7 h par jour, particulièrement au moins 8 h par jour, pendant au moins 5 jours consécutifs.

3. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon la revendication 1 ou 2, où la température corporelle centrale dudit patient est maintenue à une température comprise entre 39 °C et 40,5 °C, de préférence entre 39,5 °C et 40,5 °C, pendant au moins 5 h par jour pendant au moins 6 jours consécutifs, de préférence pendant au moins 7 jours consécutifs, particulièrement pendant au moins 8 jours consécutifs.

4. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon l'une quelconque des revendications 1 à 3, où la température corporelle dudit patient est contrôlée par l'administration d'IL-2.

5. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon l'une quelconque des revendications 1 à 4, où le traitement ne comprend pas d'administration d'un antipyrétique, particulièrement d'un médicament anti-inflammatoire non stéroïdien (AINS).

6. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon l'une quelconque des revendications 1 à 5, où l'IL-2 est administrée par une administration continue de 100 000 à 1 000 000 unités/kg de poids corporel, de préférence 400 000 à 720 000 unités/kg de poids corporel.

7. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon l'une quelconque des revendications 1 à 6, où, indépendamment les uns des autres, chaque anticorps est administré à une dose comprise entre 0,05 et 1 mg/kg de poids corporel, de préférence entre 0,1 et 0,8 mg/kg de poids corporel, particulièrement entre 0,3 et 0,5 mg/kg de poids corporel, au moins une fois par semaine, de préférence au moins deux fois par semaine, particulièrement au moins trois fois par semaine, pendant au moins deux semaines, de préférence pendant au moins trois semaines, particulièrement pendant au moins quatre semaines.

8. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon l'une quelconque des revendications 1 à 7, où du cyclophosphamide est en outre administré audit patient, de préférence en une quantité de 100 à 500 mg/m², particulièrement en une quantité de 200 à 400 mg/m².

9. (a) Anticorps qui se lie spécifiquement à et inhibe PD-1 et/ou anticorps qui se lie spécifiquement à et inhibe PD-L1 ; (b) anticorps qui se lie spécifiquement à et inhibe CTLA-4 ; et (c) IL-2 pour l'utilisation combinée selon l'une quelconque des revendications 1 à 8, où de la taurolidine est en outre administrée audit patient.
